(19) Europäisches Patentamt
European Patent Office
Office européen des brevets

(11) **EP 2 989 451 B1**

(12) **EUROPÄISCHE PATENTSCHRIFT**

(45) Veröffentlichungstag und Bekanntmachung des Hinweises auf die Patenterteilung:
**22.12.2021 Patentblatt 2021/51**

(21) Anmeldenummer: **14720571.0**

(22) Anmeldetag: **25.04.2014**

(51) Int Cl.:
**G01N 23/083** (2018.01)  **G01T 1/29** (2006.01)

(86) Internationale Anmeldenummer:
**PCT/EP2014/058458**

(87) Internationale Veröffentlichungsnummer:
**WO 2014/174077 (30.10.2014 Gazette 2014/44)**

(54) **CT-RÖNTGENPRÜFANLAGE, INSBESONDERE ZUR INSPEKTION VON OBJEKTEN**

X-RAY CT INSPECTION SYSTEM, IN PARTICULAR FOR INSPECTING OBJECTS

INSTALLATION DE CONTRÔLE DE TOMODENSITOMÉTRIE, EN PARTICULIER, POUR L'INSPECTION D'OBJETS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.04.2013 DE 102013104193**

(43) Veröffentlichungstag der Anmeldung:
**02.03.2016 Patentblatt 2016/09**

(73) Patentinhaber: **Smiths Heimann GmbH**
**65205 Wiesbaden (DE)**

(72) Erfinder:
• **HENKEL, Rainer**
**55444 Schweppenhausen (DE)**
• **HARTICK, Martin**
**61231 Bad Nauheim (DE)**

(74) Vertreter: **Klunker IP**
**Patentanwälte PartG mbB**
**Destouchesstraße 68**
**80796 München (DE)**

(56) Entgegenhaltungen:
WO-A1-2012/112153  WO-A2-2006/114716
WO-A2-2007/110795  WO-A2-2013/126649
US-A- 5 841 832  US-A1- 2013 016 805

**Beschreibung**

Gebiet der Erfindung

Die Erfindung betrifft im Allgemeinen eine Röntgenprüfanlage. Die Erfindung betrifft im Besonderen eine Röntgenprüfanlage zur zerstörungsfreien Inspektion von Objekten.

Hintergrund der Erfindung

DE 101 49 254 A1 zeigt eine Röntgenprüfanlage mit mehreren feststehenden Strahlungsebenen zur Inspektion von Objekten, beispielsweise zur Sicherheitsüberprüfung von Gepäckstücken auf Flughäfen. Das Inspektionsobjekt wird durch mehrere Strahlungsebenen gefördert, die jeweils von einer ortsfesten Strahlenquelle abgegeben werden. Die Intensitäten der nicht absorbierten Strahlen werden dabei von den Strahlenquellen zugeordneten Detektoranordnungen gemessen und ausgewertet.

US 7 362 847 B2 zeigt eine Röntgenprüfanlage der Art eines Computertomographen (CT) mit einer rotierenden Gantry der ebenfalls zur Objektinspektion verwendet wird. US 5 361 291 A zeigt ebenfalls eine CT-Vorrichtung, bei welcher zur Verdoppelung der räumlichen Abtastrate gegenüber einem herkömmlichen CT-Gerät der Brennpunkt der Röntgenröhre translatorisch bewegbar ist, um Aliasing in den rekonstruierten Röntgenbildern zu reduzieren.

EP 0 231 037 A1 zeigt einen Röntgenscanner mit einer Anordnung von Detektoren und einer Röntgenröhre mit länglicher Anode oder Doppelfokus. Der Röntgenscanner kann in verschiedenen Modi betrieben werden, wobei in einem ungepaarten Modus alle Detektorsignale getrennt ausgewertet werden, in einem gepaarten Modus Signale von zwei benachbarten Detektoren kombiniert ausgewertet werden, und in einem Dualenergie-Modus zueinander benachbarten Detektoren unterschiedliche Spektralfilter zugeordnet werden. US 7 778 383 B2 zeigt ein ähnliches CT-System zum Messen von Dual-Energie-Röntgenstrahlenabschwächungsdaten eines Objekts mit einer Röntgenquelle mit zwei verschiedenen Fokuspunkten und einer Detektionseinrichtung mit einer Vielzahl von sich in Umfangsrichtung abwechselnden ersten und zweiten Detektorelementen, wobei jeweils benachbarte Elemente eine unterschiedliche spektrale Empfindlichkeiten aufweisen. Im Betrieb wird das Objekt zuerst von Röntgenstrahlen des ersten Fokuspunkts durchstrahlt, um erste Abschwächungsdaten mit den ersten Detektorelementen und den zweiten Detektorelementen zu erfassen. Dann wird das Objekt von Röntgenstrahlen des zweiten Fokuspunkts durchstrahlt, um zweite Abschwächungsdaten mit den ersten und zweiten Detektorelementen zu erfassen. Die zwei Fokuspunkte sind räumlich voneinander so getrennt, dass ein erster Strahlengang vom ersten Fokuspunkt einen bestimmten Voxel innerhalb des Objekts durchdringt und auf ein erstes Detektorelement trifft und ein zweiter Strahlengang vom zweiten Fokuspunkt den gleichen Voxel durchdringt und auf ein benachbartes zweites Detektorelement trifft.

Die Anforderungen an Röntgenprüfanlagen zur Inspektion von Objekten, besonders zur Erkennung von Gefahrenstoffen, schreiten hinsichtlich einer Erhöhung des Gepäckdurchsatzes bei selbstverständlich gleich hoher Erkennungsrate, d.h. Detektionssicherheit, ständig voran. Andererseits sind Röntgenprüfanlagen von der Art eines Computertomographen (CT) sehr komplexe Vorrichtungen. Beispielsweise sind die in CT-Vorrichtungen benötigten Detektoreinheiten, besonders bei CTs mit Dual-Energie-Funktionalität, sehr aufwendig und aufgrund der für die angestrebten Auflösungen erforderlichen hohen Packungsdichte von Detektorelementen kompliziert und damit teuer.

WO 2007/110795 A2 zeigt CT-Verfahren und ein CT-System für die Messung von Dualenergie-Röntgenabschwächungsdaten eines Inspektionsobjekts. Das CT-System umfasst einen drehbaren Halter, eine Röntgenquelle mit zwei unterschiedlichen Röntgenfokuspunkten und eine Röntgenerfassungseinrichtung mit einer Vielzahl von ersten und zweiten Detektorelementen unterschiedlicher spektraler Empfindlichkeit, die in Drehrichtung des Halters lückenlos zueinander abwechselnd angeordnet sind. Im Betrieb des CT-Systems bzw. der Ausführung des Verfahrens werden die folgenden Schritte ausgeführt. (a) Einstellen der Röntgenstrahlenquelle, sodass sie Röntgenstrahlen von einem ersten Brennpunkt emittiert; (b) separates Erfassen erster Abschwächungsdaten sowohl mit den ersten Detektorelementen als auch mit den zweiten Detektorelementen; (c) diskretes Bewegen des Röntgenfokus an einen zweiten Fokuspunkt; und (d) separates Erfassen zweiter Abschwächungsdaten sowohl mit den ersten Detektorelementen als auch mit den zweiten Detektorelementen. Der erste und der zweite Fokuspunkte sind räumlich so voneinander getrennt, dass ein erster Strahlengang ausgehend von dem ersten Fokuspunkt ein bestimmtes Voxel innerhalb des Objekts durchläuft und auf ein erstes Detektorelement trifft, während ein zweiter Strahlengang ausgehend von dem zweiten Fokuspunkt das gleiche Voxel durchdringt und auf ein zweites Detektorelement trifft.

WO 2012/112153 A1 zeigt ein CT-Verfahren und CT-Systemen mit einer Detektoranordnung, die eine effektive Größe aufweist, die größer ist als die tatsächliche Größe der Detektorelemente der Detektoranordnung. Dazu wird ein sogenannter Sparse-Array vorgeschlagen, bei dem ein oder mehrere Kanäle des Detektorarrays weglassen sind, sodass sich zwischen den Detektorelementen in Drehrichtung der Detektoranordnung Lücken befinden. Ähnlich zur WO 2012/112153 A1 zeigen WO 2012/112153 A1 und WO 2013/126649 A2 ebenfalls Detektoranordnung mit Sparse-Arrays.

US 5 841 832 A und WO 2006/114716 A2 zeigen Dual-Energie-Röntgengeräte, die sogenannte Dual-Energie-Röntgendetektoren aufweisen, in denen zwei Arten von Detektorelementen, die jeweils für die Detektion von Röntgenstrahlen hoher oder niedriger Energie ausgelegt sind, neben oder übereinander angeordnet sind.

Zusammenfassung der Erfindung

Es ist eine Aufgabe der vorliegenden Erfindung, eine Röntgenprüfanlage für die Detektion von Materialien, insbesondere Gefahrenstoffen, in einem Objekt, insbesondere einem Gepäckstück, vorzuschlagen, die eine Vereinfachung beim apparativen Aufwand erreicht. Es ist insbesondere eine Aufgabe der Erfindung, trotz geringeren apparativen Aufwands eine geforderte Detektionssicherheit zu erreichen.

Diese Aufgabe wird mit den Merkmalen einer Röntgenprüfanlage gemäß dem unabhängigen Anspruch 1 gelöst. Weitere Merkmale und Details der Erfindung ergeben sich aus den Unteransprüchen, der Beschreibung und den Zeichnungen. Dabei gelten Merkmale und Details, die im Zusammenhang mit der erfindungsgemäßen Röntgenprüfanlage beschrieben sind, auch im Zusammenhang mit dem erfindungsgemäßen Verfahren gemäß dem unabhängigen Anspruch 5 und jeweils umgekehrt, sodass bezüglich der Offenbarung der einzelnen Erfindungsaspekte stets wechselseitig Bezug genommen wird bzw. werden kann.

Ein Kerngedanke der Erfindung liegt in der Erkenntnis, bei einer Röntgenprüfanlage mit rotierender Strahlungsquelle und mit (i) einer gegenüberliegend mitrotierend angeordneten Detektoreinheit oder mit (ii) einer feststehend um die von der rotierender Strahlungsquelle beschriebenen Kreisbahn herum angeordneten ringförmigen Detektoreinheit, durch eine Erhöhung der Anzahl der Strahlungsquellen, beispielsweise der Röntgenbrennpunkte (Foci), die Anzahl der benötigten Detektorelemente in Umfangsrichtung der Rotation zu verringern. Dadurch kann der Füllfaktor (Verhältnis der sensitiven Fläche zur Gesamtfläche) der Detektoreinheit und/oder der Detektorelemente gegenüber den bekannten Röntgenprüfanlagen reduziert werden. Beispielsweise ermöglicht eine Erhöhung der Strahlungsquellen von eins auf zwei, d.h. eine Verdopplung, wenigstens eine Halbierung der Anzahl der benötigten Detektorelemente an der Detektoreinheit in Rotationsrichtung.

Die Erfinder haben zunächst erkannt, dass wenn der Abstand (in Rotationsrichtung) zwischen den wenigstens zwei Strahlungsquellen in der Größenordnung der Breite eines Detektorelements eingestellt wird, der Abstand zwischen den Detektorelementen in Umfangrichtung der Rotation ohne Auswirkungen auf die Auflösung auf wenigstens die Breite eines Detektorelements erhöht werden kann. Mit anderen Worten kann jedes zweite Detektorelement in Wegfall gebracht werden. Infolgedessen kann vorteilhaft aufgrund des freiwerdenden Raums in der Detektoreinheit der Aufbau der Detektorelemente bzw. der Detektoreinheit vereinfacht werden. Zwischen zwei benachbarten Detektorelementen befindet sich dann eine Lücke mit der Breite eines Detektorelements. Diese Lücke steht zusätzlich beispielsweise für die Verdrahtung der Detektorelemente zur Verfügung. Im Ergebnis sind mit der so vereinfachten Röntgenprüfanlage Röntgenaufnahmen realisierbar, welche die gleiche Qualität, insbesondere die gleiche Auflösung aufweisen wie Röntgenaufnahmen, die mit einer vergleichbaren Röntgenprüfanlage mit nur einer Strahlungsquelle und der üblichen hohen Anzahl von Detektorelementen, die in Umfangsrichtung lückenlos auf der Detektoreinheit angeordnet sind, angefertigt wurden.

Die Erfinder haben weiter erkannt, dass wenn der Abstand zwischen den wenigstens zwei Strahlungsquellen in der Größenordnung der Breite eines Detektorelements eingestellt wird, der Abstand zwischen den Detektorelementen in Umfangrichtung der Rotation auf bis zu der doppelten, ggf. bis auf die 2,5-fachen Breite eines Detektorelements erhöht werden kann. Diese Vergrößerung der Lücken hat dann jedoch eine Verringerung bei der Qualität der Röntgenaufnahmen zur Folge. Dies bedeutet, dass die Lückenbreite in dem angegebenen Umfang im Hinblick auf eine geforderte Qualität, insbesondere Auflösung, der Röntgenbilder, entsprechend eingestellt werden kann. Im Ergebnis lassen sich sogar mehr als die Hälfte der Detektorelemente einer Detektoreinheit mit 100% Füllfaktor einsparen. D.h., mit dem Mehraufwand für die zweite oder weitere Röntgenstrahlungsquellen bzw. Foci kann eine entsprechende Reduzierung des Aufwands bei der Detektoreinheit neben zusätzlichen Vorteilen erreicht werden. Damit ist eine erfindungsgemäße Röntgenprüfanlage kostengünstiger.

Eine erfindungsgemäße Röntgenprüfanlage zur zerstörungsfreien Inspektion von Objekten kann somit aufweisen: wenigstens zwei um eine Rotationsachse in einer Rotationsebene drehbare Röntgenstrahlungsquellen (auch Foci bzw. Brennflecke genannt) zum Erzeugen jeweils eines einen Untersuchungsbereich durchsetzenden Strahlenbündels und eine der wenigstens einen Röntgenstrahlungsquelle gegenüberliegend angeordnete Detektoreinheit mit Detektorelementen für Röntgenstrahlen. Ein Inspektionsobjekt kann mittels einer Transporteinrichtung entlang einer Förderrichtung durch den Untersuchungsbereich gefördert werden, wobei die Förderrichtung für das Inspektionsobjekt im Wesentlichen senkrecht zu der Rotationsebene verläuft.

Zueinander in Umfangsrichtung der Rotation der Röntgenstrahlungsquellen benachbarte Detektorelemente mit einem ersten Abstand D1 so beabstandet, dass sie eine Lücke bilden; d.h., der Füllfaktor der Detektoreinheit ist damit kleiner als 100%.

Die wenigstens zwei unabhängig ansteuerbaren Röntgenstrahlungsquellen (Foci) sind in Umfangrichtung in der Rotationsebene mit einem zweiten Abstand D2 beabstandet angeordnet. Bevorzugt sind die wenigstens zwei unabhängig ansteuerbaren Röntgenstrahlungsquellen so beabstandet, dass sich entlang einer ersten Geraden, die (a) durch das Rotationszentrum der Foci und (b) senkrecht zu einer zweiten Geraden verläuft, die (i) von dem Punkt auf der Rotationsbahn der Foci, der in der Mitte zwischen den beiden Foci liegt, und (ii) durch das Rotationszentrum der Foci verläuft, wenigstens in einem Abschnitt um das Rotationszentrum eine gewünschte räumliche Auflösung durch die Kombination der beiden Foci mit den beabstandeten Detektorelementen der Detektoreinheit ergibt. D.h., dieser Abschnitt der ersten Gerade entspricht im Wesentlichen dem Untersuchungsbereich oder Messfeld der Röntgenprüfanlage. Mit anderen Worten kann der das Messfeld bildende Abschnitt der ersten Geraden als virtueller Detektor interpretiert werden, der die benötigte bzw. geforderte räumliche Auflösung aufweist. Aufgrund der Lücken zwischen den Detektorelementen wird der bei CT-Anlagen zunächst ein systembedingter Effekt zusätzlich verstärkt, nämlich dass im Halbkreis der Foci über dem virtuellen Detektor zu den Foci hin eine Überabtastung und im Halbkreis unter dem virtuellen Detektor zu den Detektorelementen hin eine Unterabtastung erfolgt. Da sich die Anordnung aus den wenigstens zwei Röntgenstrahlungsquellen und der Detektoreinheit um das Inspektionsobjekt herum dreht, gleichen sich die Unter- und Überabtastung aus und es wird für das Inspektionsobjekt die Auflösung des virtuellen Detektors erreicht.

Das Verhältnis D1/B des ersten Abstands D1 zu einer Breite B eines ersten Detektorelements in Umfangsrichtung beträgt aufgrund der Lücken mehr als 100% und höchstens 250%. D.h., die Lücke ist größer als die Breite B und maximal das 2,5-fache der Breite B eines Detektorelements. Das Verhältnis D1/B des ersten Abstands D1 zu der Breite B eines ersten Detektorelements in Umfangsrichtung kann in bestimmten Ausführungen mindestens mehr als 110%, oder mehr als 125%, oder mehr als 150%, oder mehr als 175% oder mehr als 200% betragen.

Wie bereits erläutert, vereinfacht sich durch die Lücken der Aufbau der Detektoreinheit. Dies gilt auch für die Detektorelemente. Beispielsweise können die Lücken zwischen den Detektorspalten zur Verdrahtung der Detektorelemente genutzt werden. Da für die Detektorelemente in eine Detektorspalte aufgrund der Lücken mehr Platz zur Verfügung steht, können die Detektorelemente mit der Detektionsfläche besser in Richtung der Foci ausgerichtet werden, indem die Detektionsfläche gegenüber einer Tangente an die Rotationsbahn zu den Foci hin geneigt angeordnet ist. Ebenso reduzieren sich auch Anforderungen an die Präzision für andere Teile der Anlage. Beispielsweise muss ein Röntgenraster bzw. Streustrahlenraster (anti-scatter grid, Bucky-Blende), das vor der Detektoreinheit angebracht wird, um den Einfall von Streustrahlung auf die Detektorelemente zu reduzieren. Mit dieser Maßnahme wird der Kontrast des Röntgenbildes erhöht. Das Streustrahlenraster entspricht im Prinzip den Kollimatoren an der Röntgenstrahlenquelle. Aufgrund der Lücken zwischen den Detektorelementen vereinfacht sich die Fertigung des Streustrahlenrasters entsprechend.

Unter Röntgenstrahlungsquelle wird hier ein Punkt verstanden, von dem erzeugte Röntgenstrahlen bestimmungsgemäß ausgehen. D.h., Röntgenstrahlungsquelle steht hier synonym für Röntgenstrahlen-Focus (kurz Focus) oder Röntgenstrahlen-Brennfleck (kurz: Brennfleck). Die eigentliche Erzeugung der Röntgenstrahlen erfolgt in einer Röntgenstrahlenerzeugungseinrichtung, wie beispielsweise einer Röntgenröhre. D.h., die erfindungsgemäßen wenigstens zwei unabhängig aktivierbaren Röntgenstrahlungsquellen (Foci bzw. Brennflecke) können eine gemeinsame Röntgenstrahlenerzeugungseinrichtung aufweisen. Es können aber auch zwei unabhängig ansteuerbare bzw. aktivierbare Röntgenstrahlungserzeugungseinrichtungen, d.h. für jeden Focus bzw. Brennfleck eine eigene Röntgenstrahlenerzeugungseinrichtung, vorgesehen sein. Entsprechend ist klar, dass für eine Ausführung mit z.B. vier unabhängig aktivierbaren Foci bzw. Brennflecken, eine Röntgenstrahlungserzeugungseinrichtung mit vier unabhängig aktivierbaren Foci, oder zwei Röntgenstrahlungserzeugungseinrichtungen mit jeweils zwei unabhängig aktivierbaren Foci, oder auch vier unabhängig aktivierbare Röntgenstrahlungserzeugungseinrichtungen verwendet werden können.

Dadurch, dass erfindungsgemäß wenigstens eine zweite Röntgenstrahlungsquelle (Focus/Brennfleck) vorgesehen ist, werden bei gleicher Detektionsauflösung der Anlage, aufgrund der Lücken zwischen den Detektorelementen insgesamt weniger Detektionselemente benötigt. Dadurch vereinfacht sich der Aufwand für die Detektoreinheit entsprechend. Zusätzlich vereinfacht sich der Aufbau der Detektoreinheit, da bei einem deutlich kleineren physikalischen Füllfaktor im Wesentlichen ein gleicher effektiver Füllfaktor (bei der virtuellen Detektoreinheit) erreicht wird, wie bei einer herkömmlichen Detektoreinheit, bei der der Füllfaktor annähernd 100% beträgt. Die mögliche Reduktion beim Aufwand hinsichtlich der Detektoreinheit kompensiert den Mehraufwand durch die wenigstens zweite Röntgenstrahlungsquelle bei Weitem. Es ist davon auszugehen, dass Kostenvorteile wenigstens im Bereich von 2 bis 4 Röntgenstrahlungsquellen erzielt werden können.

Erfindungsgemäß sind die ersten Detektorelemente auf einer ersten Kreisbahn mit einem ersten Radius R1' angeordnet. Die wenigstens zwei unabhängig ansteuerbaren Röntgenstrahlungsquellen sind in der Rotationsebene auf einer zweiten Kreisbahn mit einem zweiten Radius R2 um die Rotationsachse angeordnet, wobei die erste Kreisbahn um einen Punkt auf der zweiten Kreisbahn mittig zu den wenigstens zwei unabhängig ansteuerbaren Röntgenstrahlungsquellen angeordnet ist. Für den zweiten Abstand D2 zwischen den wenigstens zwei unabhängig ansteuerbaren Röntgenstrahlungsquellen gilt dann:

$$D2 \leq 2\frac{R1'+R2}{2R1'}B. \qquad (1)$$

In bestimmten Ausführungen, beispielsweise in solchen, bei denen die Röntgenprüfanlage eine um den Untersuchungsbereich drehbare Gantry aufweist, können die Detektorelemente und die wenigstens zwei Röntgenstrahlungsquellen im Wesentlichen auf derselben Kreisbahn angeordnet sein, sodass der erste Radius R1 gleich dem zweiten Radius R2 ist bzw. der erste Radius R1' ungefähr doppelt so groß wie der zweite Radius R2 ist.

Grundsätzlich hat es sich bewährt, den zweiten Abstand D2 zwischen den wenigstens zwei Röntgenstrahlungsquellen bevorzugt ungefähr gleich oder kleiner der Breite B eines Detektorelements einzustellen. Der zweite Abstand D2 wird bevorzugt so eingestellt, dass der oben erläuterte, das Messfeld bildende virtuelle Detektor die geforderte räumliche Auflösung aufweist.

Erfindungsgemäß bestehen die Detektorelemente aus ersten Detektorelementen, die für die Detektion von Röntgenstrahlung in einem ersten Energiebereich (bzw. Frequenzbereich) eingerichtet sind. Weiter sind zweite Detektorelementen, die für die Detektion von Röntgenstrahlung in einem zweiten Energiebereich (bzw. Frequenzbereich) eingerichtet sind, vorgesehen. Die zweiten Detektorelemente haben bevorzugt dieselbe Breite B wie die ersten Detektorelemente.

Dabei ist - mit Bezug auf den Strahlengang - unter jeweils einem ersten Detektorelement jeweils eines der zweiten Detektorelemente angeordnet. Bevorzugt sind dabei die ersten Detektorelemente für Röntgenstrahlen aus einem niedrigeren Energiebereich ausgelegt und die darunter angeordneten zweiten Detektorelemente für Röntgenstrahlen aus einem höheren Energiebereich ausgelegt. Mit einer solchen Detektoranordnung kann eine Mehrenergie- bzw. Dual-Energy-Auswertung erfolgen.

Bei den zwei unterschiedlichen Arten von Detektorelementen ist besonders vorteilhaft, dass die Verdrahtung keines der Detektorelemente im Strahlengang eines anderen Detektorelements angeordnet werden muss. Dies vereinfacht die Verdrahtung der Detektorelemente der Detektoreinheit deutlich, da für die Verdrahtung zusätzlich die Lücke genutzt werden kann.

Mit der Röntgenprüfanlage der vorstehenden Weiterbildungen kann eine Mehrenergie- bzw. Dual-Energy-Auswertung erfolgen, wodurch sich für einzelne Raumelemente des Inspektionsobjekts die effektive Kernzahl (Zeff) des dort befindlichen Materials bestimmen lässt.

Die Detektorelemente sind in der Detektionseinheit in Richtung der Rotationsachse bzw. in Förderrichtung des Inspektionsobjekts jeweils in Reihen mit mehreren Detektorelementen als Detektorspalte angeordnet.

Weiter wurde festgestellt, dass, wenn die Röntgenprüfanlage eine Anzahl n, mit n größer gleich 2, unabhängig ansteuerbarer Röntgenstrahlungsquellen aufweist, es von Vorteil ist, die Prüfanlage so einzurichten, dass die n unabhängig ansteuerbaren Röntgenstrahlungsquellen im Betrieb der Röntgenprüfanlage abwechselnd bzw. nacheinander bzw. der Reihe nach mit wenigstens der n-fachen Frequenz angesteuert werden als dies bei gleicher Rotationsgeschwindigkeit an einer Röntgenprüfanlage mit nur einer einzigen Röntgenstrahlungsquelle erfolgt. Alternativ kann die Frequenz reduziert werden, wenn die Rotationsgeschwindigkeit entsprechend reduziert wird. Dann können in bestimmten Ausführungen Röntgenbilder mit im Wesentlichen der gleichen Auflösung rekonstruieren werden. Entsprechend werden die Detektorelemente ebenfalls mit der n-fachen Frequenz ausgelesen.

Weiter wurde festgestellt, dass, wenn die Röntgenprüfanlage eine Anzahl n, mit n größer gleich 2, unabhängig ansteuerbarer Röntgenstrahlungsquellen aufweist, es von Vorteil ist, die Prüfanlage so einzurichten, dass die n unabhängig ansteuerbaren Röntgenstrahlungsquellen jeweils Röntgenstrahlen mit einer wenigstens n-fach größeren Strahlungsintensität abgeben als dies bei gleicher Rotationsgeschwindigkeit in einer Röntgenprüfanlage mit einer einzigen Röntgenstrahlungsquelle erfolgt.

Die Röntgenprüfanlage kann weiter eine Transporteinrichtung aufweisen, welche zumindest teilweise in einer Einhausung der Röntgenprüfanlage angeordnet und eingerichtet ist, ein Inspektionsobjekt durch die Röntgenprüfanlage hindurch in Förderrichtung im Wesentlichen senkrecht zur Rotationsebene der Röntgenstrahlungsquellen zu transportieren. Die Transporteinrichtung kann eingerichtet sein, ein Inspektionsobjekt kontinuierlich oder schrittweise durch den Untersuchungsbereich zu transportieren.

Die Röntgenprüfanlage kann weiter aufweisen einen feststehenden Teil, in dem drehbar um eine Rotationsachse eine Gantry gelagert ist. Dabei können an der Gantry wenigstens die wenigstens zwei Röntgenstrahlungsquellen in der Rotationsebene angeordnet sein, die dann mit der Gantry um die Rotationsachse drehbar sind. Die Detektoreinheit kann den wenigstens zwei Röntgenstrahlungsquellen gegenüberliegend ebenfalls um die Rotationsachse drehbar an der Gantry angeordnet sein, d.h., die Detektoreinheit wird ebenfalls um den Untersuchungsbereich mit fester Anordnung gegenüber den wenigstens zwei Röntgenstrahlungsquellen gedreht. Bei dieser Ausführung muss die Detektoreinheit nur einen Teilabschnitt des Rotationskreises abdecken, wodurch entsprechend wenige Detektorelemente benötigt werden.

Es ist aber auch möglich, die Detektoreinheit so auszuführen, dass diese den Untersuchungsbereich in Umfangsrichtung umfassend, d.h. ringförmig, an dem feststehenden Teil angeordnet ist. Bei dieser Ausführung ist die Detektoreinheit zwar aufwendiger in Bezug auf die Anzahl der Detektorelemente, aber die Ansteuerung und Verdrahtung vereinfacht sich dadurch, dass die Detektoreinheit nicht (mit)drehend (d.h. feststehend) an dem feststehenden Teil der Prüfanlage angeordnet werden kann.

Ein erfindungsgemäßes Verfahren zur zerstörungsfreien Inspektion eines Objekts mit einer Röntgenprüfanlage gemäß einer der vorstehend diskutierten Ausführungen, weist auf: Drehen wenigstens zweier unabhängig ansteuerbarer Röntgenstrahlungsquellen in einer Rotationsebene um einen Untersuchungsbereich; abwechselndes Aktivieren jeder der zwei unabhängig ansteuerbaren Röntgenstrahlungsquellen und dabei jeweils Bestrahlen von einer den Röntgenstrahlungsquellen gegenüberliegend angeordneten Detektoreinheit mit Detektorelementen durch den Untersuchungsbereich hindurch; Transportieren eines Inspektionsobjekts durch den Untersuchungsbereich, und Erfassen und Auswerten der Intensitäten von das Inspektionsobjekt durchdringender Röntgenstrahlung mittels erster und zweiter Detektorelemente.

Die Erfindung eignet sich besonders für Röntgenprüfanlagen, die an Sicherheitsüberprüfungsstellen im Gepäckumschlagsystem, beispielsweise an Flughäfen, eingesetzt werden, um Gepäckstücke und Frachtgüter, die an Bord eines Flugzeugs verbracht werden sollen, automatisch zerstörungsfrei zu inspizieren. Die Anlage kann auch an anderen Kontrollstellen z.B. an Zugängen zu sicherheitsrelevanten Bereichen oder Gebäuden, an Grenzkontrollstellen etc. zur Inspektion von Objekten, wie von Personen mitgeführtes Handgepäck oder Poststücke wie Briefe, Päckchen und Pakete verwendet werden. Ziel derartiger Inspektionen kann die Entdeckung bestimmter Gegenstände oder Stoffe, wie beispielsweise Waffen, Schmuggelgüter, Drogen, Geld und dergleichen, aber auch die Detektion von Materialen und Substanzen mit Gefährdungspotenzial, wie Sprengstoffe oder Chemikalien, sein. Auch die Detektion von Lebensmitteln ist denkbar.

Ein weiteres Einsatzgebiet für derartige Röntgenprüfanlagen ist die zerstörungsfreie Materialprüfung zur Erkennung äußerlich nicht sichtbarer Fehler, wie Materialschwächen, Bruchstellen, Lunker, Einschlüsse, Porositäten, Formabweichungen oder Ahnliches. Beispielsweise sind Turbinenschaufeln moderner Triebwerke mit Kanälen oder Kanalsystemen versehen, durch die während des Betriebes Kühlluft geblasen wird. Mittels Röntgeninspektion können solche Turbinenschaufeln hinsichtlich Wanddicke und Fehler im Kühlsystem geprüft werden, um Beschädigungen, Einschlüsse, Risse, Bruchstellen, Verstopfungen und Unterbrechungen des Kanalsystems zu erkennen. Ein weiteres Anwendungsbeispiel ist die Prüfung der Qualität von Schweißnähten, dabei können Lunker, Risse, Löcher, Unterbrechungen, Gefügefehler, Längs- und Querschnittsfehler, ungenügende Durchschweißung, Einschlüsse erkannt werden. Auch ein Einsatz zur Qualitätssicherung bei komplexen elektrischen Bauelementen ist möglich, um z.B. elektrische Bauteile auf Vollständigkeit und korrekte Montage der Einzelteile zu kontrollieren, dabei können von außen nicht erkennbare Kabelbrüche, defekte Anschlüsse, fehlende Bauteile, falsche Lage der Bauteile, Montagefehler, Brüche in Federteilen, fehlerhafte Lötstellen oder dergleichen erkannt werden. Ebenso sind bei mikroelektronischen Produkten die Erkennung feinster Bonddrähte und somit defekte Bondungen, gerissene Bonddrähte, Bruchstellen in einem Chip, fehlerhafte Chip-Verklebung etc. möglich.

Schließlich kann die Röntgenprüfanlage auch zur Gewährleistung der Produktsicherheit für die Detektion von Fremdkörpern an verschiedenen Stellen einer Produktionslinie eingesetzt werden. Ein mögliches Beispiel hierfür ist die Detektion von Glasscherben in Lebensmitteln.

Kurzbeschreibung der Zeichnungsfiguren

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung im Einzelnen beschrieben sind. Dabei können die in den Ansprüchen und in der Beschreibung erwähnten Merkmale jeweils einzeln für sich oder in beliebiger Kombination erfindungswesentlich sein. Funktionsähnliche oder identische Bauteile oder Komponenten sind teilweise mit gleichen Bezugszeichen versehen. Die in der Beschreibung der Ausführungsbeispiele verwendeten Begriffe "links", "rechts", "oben", "unten" beziehen sich auf die Zeichnungen in einer Ausrichtung mit normal lesbaren Figurenbezeichnungen bzw. Bezugszeichen. Hierbei zeigen (schematisch):

Figur 1       eine Röntgenprüfanlage gemäß der Erfindung;

Figur 2       eine Draufsicht auf eine erste Ausführung einer erfindungsgemäßen Detektoreinheit für die Röntgenprüfanlage der Figur 1;

Figur 3a      eine schematische Draufsicht auf die Gantry der Röntgenprüfanlage der Figur 1 mit Blickrichtung entlang der Rotationsachse und der Detektoreinheit der Figur 2;

Figur 3b      eine schematische Draufsicht auf die Gantry der Röntgenprüfanlage der Figur 1 mit Blickrichtung entlang der Rotationsachse und einer Abwandlung der Detektoreinheit der Figur 3a;

Figur 4       eine Weiterbildung der Detektoreinheit der Figur 3 mit ersten und zweiten übereinander angeordneten Detektorelementen für eine Mehrenergie-Auswertung;

Figur 5    eine Draufsicht auf ein Beispiel, welches nicht Teil der Erfindung ist, mit einer Detektoreinheit für eine Mehrenergie-Auswertung mit ersten und zweiten nebeneinander angeordneten Detektorelementen;

Figur 6    eine schematische Draufsicht auf die Gantry einer beispielhaften Modifikation der Röntgenprüfanlage der Figur 1, welche nicht Teil der Erfindung ist, mit Blickrichtung entlang der Rotationsachse und der der Detektoreinheit der Figur 5; und

Figur 7    ein Blockschaltbild eines Datenverarbeitungssystems zur erfindungsgemäßen Verarbeitung von erfassten Intensitätswerten für das Inspektionsobjekt durchdringender Röntgenstrahlen.

Detaillierte Beschreibung der Ausführungsbeispiele

In der nachfolgenden Beschreibung werden zahlreiche spezifische Einzelheiten der Erfindung dargelegt. Es versteht sich jedoch, dass weitere Ausführungsformen auch ohne diese spezifischen Einzelheiten möglich sind. Dem Fachmann bekannte Schaltungen, Strukturen und Verfahren werden hier nicht im Detail besprochen, um das Verständnis der vorliegenden Beschreibung nicht unnötig zu erschweren. Begriffe "gekoppelt" und "verbunden/angeschlossen" sowie davon abgeleitete Begriffe werden hier nicht synonym verwendet. Bei spezifischen Ausführungsformen kann vielmehr "verbunden/angeschlossen" andeuten, dass zwei oder mehr Elemente in direktem physischem oder elektrischem Kontakt miteinander stehen. "Gekoppelt" kann bedeuten, dass zwei oder mehr Elemente zusammenwirken oder sich gegenseitig beeinflussen, wobei sie in direktem, aber auch indirektem physischem oder elektrischem Kontakt miteinander stehen können. Falls nicht anderweitig angegeben, deutet der Gebrauch der Ordinaladjektive "erster", "zweiter", "dritter" usw. zur Bezeichnung eines gemeinsamen Objekts lediglich an, dass auf verschiedene Beispiele von gleichartigen Objekten Bezug genommen wird, und soll nicht implizieren, dass die so bezeichneten Objekte in einer gewissen zeitlichen, räumlichen, Rangordnungs- oder sonstigen Reihenfolge auftreten müssen.

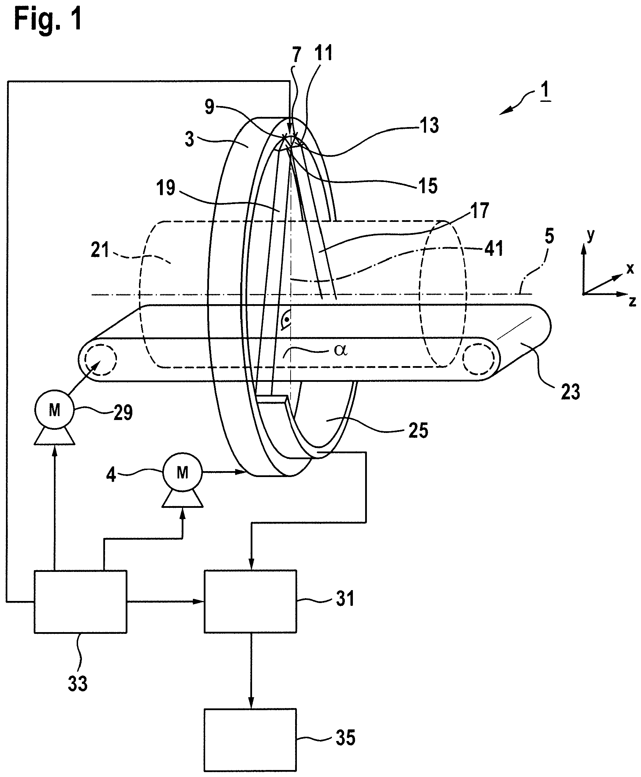

Figur 1 zeigt schematisch eine Röntgenprüfanlage 1 mit einer Gantry 3, die um eine Rotationsachse 5 rotierbar an einem feststehenden Teil (nicht gezeigt) der Anlage gelagert ist. Im Betrieb der Anlage wird die Gantry 3 von einem Motor 4 mit einer vorzugsweise konstanten, aber einstellbaren Winkelgeschwindigkeit angetrieben. An der Gantry 3 befinden sich zwei (Röntgen-)Strahlenquellen bzw. zwei Foci 7, 9; wie oben erläutert kann für die beiden (Röntgen-)Strahlenquellen jeweils eine eigene oder eine gemeinsame Röntgenstrahlungserzeugungseinrichtung vorgesehen sein.

Als (Röntgen-)Strahlungserzeugungseinrichtung(en) können Röntgenstrahler, beispielsweise in Form separater bzw. einzelner Röntgenröhren bevorzugt mit einer Stehanode oder auch mit einer rotierenden Anode (z.B. Drehanodenröhren oder Drehkolbenröhren), eingesetzt werden. Es ist auch möglich, für die zwei Foci 7, 9 zwei Röntgenstrahler in einer einzigen Röntgenröhre anzuordnen, die dann beispielsweise so eingerichtet ist, dass zwischen zwei Anoden-Brennpunkten bzw. Foci geschaltet werden kann. Die Röntgenröhre(n) als Röntgenstrahlungserzeugungseinrichtung(en) ist (bzw. sind) im dargestellten Ausführungsbeispiel in einem Strahlergehäuse 11 an der Gantry 3 untergebracht, d.h. die Röntgenstrahlungserzeugungseinrichtung(en) werden mitgedreht.

Die Strahlungsquellen 7, 9 sind weiter jeweils mit einer Kollimatoranordnung 13, 15 ausgestattet, die aus von der jeweiligen Strahlungsquelle 7, 9 erzeugten Strahlung ein kegelförmiges oder fächerförmiges Strahlenbündel 17, 19 ausblendet; ein kegelförmiges Strahlenbündel hat sowohl in einer zur Rotationsachse 5 senkrechten Ebene als auch in Richtung der Rotationsachse 5 eine von Null verschiedene endliche Ausdehnung. Demgegenüber hat ein fächerförmiges Strahlenbündel im Wesentlichen in der zur Rotationsachse 5 (Rotationszentrum) senkrechten Ebene eine von Null verschiedene endliche Ausdehnung und in Richtung der Rotationsachse 5 eine relativ kleine Ausdehnung.

Weiter sind in der Gantry 3 noch weitere notwendige (hier aus Vereinfachungsgründen nicht dargestellte) Peripheriegeräte und Aggregate angeordnet.

Die Strahlenbündel 17, 19 durchdringen einen Untersuchungsbereich 21, in dem sich ein zu inspizierendes Objekt (Inspektionsobjekt), beispielsweise ohne Anspruch auf Vollständigkeit ein Gepäckstück bzw. Frachtgut zum Auffinden von Gefahrenstoffen oder Schmuggelgut und dergleichen oder auch ein Werkstück oder Produkt zur Qualitätskontrolle und dergleichen, auf einer Transporteinrichtung 23, wie beispielsweise einem Transportband, befinden kann.

Zur besseren Orientierung ist in Figur 1 ein xyz-Koordinatensystem definiert. Die z-Richtung entspricht der Förderrichtung der Transporteinrichtung 23. Die x-Richtung verläuft quer zur Förderrichtung und parallel zur Förderebene der Transporteinrichtung 23. Die y-Richtung verläuft jeweils orthogonal zur z- bzw. x-Richtung und bei normaler Aufstellung der Röntgenprüfanlage 1 senkrecht zur Aufstellfläche.

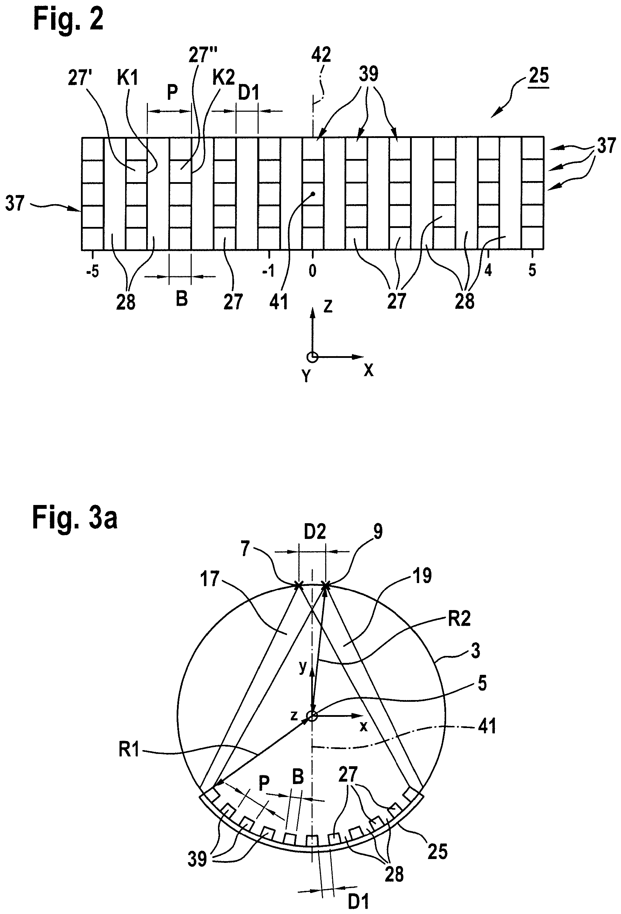

Der Untersuchungsbereich 21 ist im Wesentlichen zylinderförmig, wobei die Längsachse des Zylinders der Rotationsachse 5 der Gantry 3 entspricht. Nach der Durchdringung dieses Untersuchungsbereichs 21, mithin eines zu inspizierenden Objekts, treffen die Röntgenstrahlen der Strahlenbündel 17, 19 auf eine ebenfalls an der Gantry 3 befestigte Detektoreinheit 25, die eine Vielzahl von Detektorelementen 27 (vgl. Figuren 2 bis 6) aufweist.

Jedes Detektorelement 27 kann in jeder Position der Strahlenquellen 7, 9 jeweils einen Messwert für einen (Röntgen-)Strahl aus jeweils einem der Strahlenbündel 17, 19 liefern. Hierzu werden die zwei Strahlungsquellen 7, 9 getrennt voneinander angesteuert, d.h. unabhängig voneinander aktiviert. Mit anderen Worten werden die Strahlungsquellen abwechselnd aktiviert, sodass immer ein durch ein Detektorelement 27 der Detektoreinheit erfasster Messwert genau bzw. eindeutig einer der Strahlungsquellen 7, 9 zugeordnet werden kann.

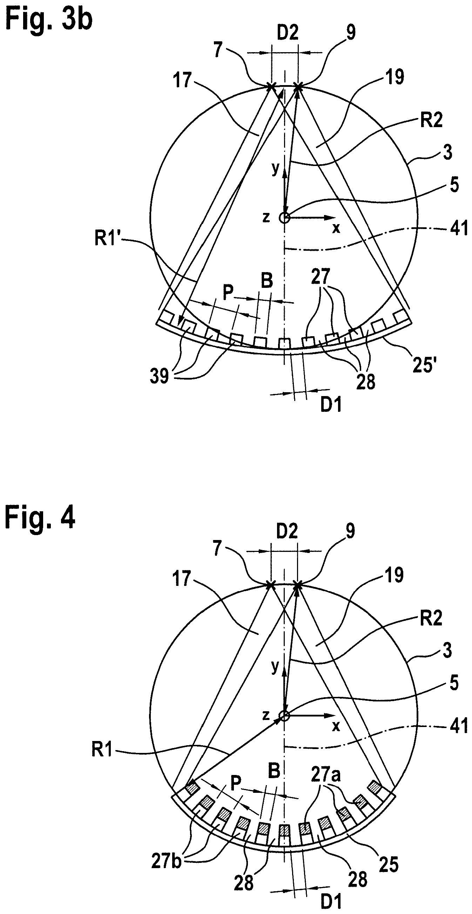

Wie anhand der Figuren 2 bis 6 genauer veranschaulicht, sind die Detektorelemente 27 in Detektorzeilen und Detektorspalten angeordnet. Eine Detektorzeile befindet sich in einer zur Rotationsachse 5 senkrechten (Rotations-)Ebene, auf einem Kreisbogen um die Rotationsachse 5 (vgl. Figur 3a, 4 und 6) bzw. auf einem Kreisbogen im Wesentlichen annähernd um eine der oder einen Punkt zwischen den Strahlenquellen 7, 9 (vgl. Figur 3b), d.h., eine Detektorzeile verläuft in Umfangsrichtung. Detektorspalten verlaufen parallel zur Rotationsachse 5, d.h. in z-Richtung. Die Detektorzeilen enthalten üblicherweise wesentlich mehr Detektorelemente als die Detektorspalten. Beispielsweise können die Detektorelemente solche wie die aus der EP 1 186 909 bekannte Elemente sein.

Der mit $\alpha$ in Figur 1 angetragene Öffnungswinkel der Strahlenbündel 17, 19 ist hier im Wesentlichen der Winkel, den ein Strahl, der in einer zur Rotationsachse 5 senkrechten Ebene am Rande des Strahlenbündels 17, 19 liegt, mit einer durch einen Punkt zwischen den Strahlenquellen 7, 9 und die Rotationsachse 5 definierten Ebene einschließt. Der Öffnungswinkel $\alpha$ ist im Wesentlichen bestimmend für den Durchmesser des Untersuchungsbereichs 21 (Messfeld), innerhalb dessen sich das Inspektionsobjekt bei der Akquisition der Messwerte befinden sollte, da dort im Wesentlichen die durch den Abstand D2 zwischen den Strahlenquellen 7, 9 und den Abstand D1 zwischen zwei benachbarten Detektorelementen 27 definierte räumliche Auslösung (des virtuellen Detektors) erreicht wird.

Ein Inspektionsobjekt wird mittels der durch einen Motor 29 angetriebenen Transporteinrichtung 23 parallel zur und in Richtung der Rotationsachse 5, d.h. in z-Richtung durch den Untersuchungsbereich 21 transportiert. Die Geschwindigkeit des Vorschubs ist vorzugsweise konstant und einstellbar. Wenn der die Gantry 3 antreibende Motor 4 und der die Transporteinrichtung 23 antreibende Motor 29 gleichzeitig laufen, ergibt sich hinsichtlich der Auswertung der Detektorelemente 27 der Detektoreinheit 25 ein Helix-förmiger Verlauf der Abtastbewegung der Strahlungsquellen 7,9 durch die Detektoreinheit 25. Selbstverständlich wäre es auch möglich, dass der Motor für den Vorschub der Transporteinrichtung 23 in Richtung der Rotationsachse 5 (in z-Richtung) immer für die Dauer der Erstellung eines oder mehrerer Schnittbilder des Inspektionsobjekts stillsteht, während die Gantry 3 gedreht wird. Dann ergibt sich hinsichtlich der Auswertung der Detektorelemente der Detektoreinheit 25 ein kreisförmiger Verlauf der Abtastbewegung der Strahlungsquellen 7, 9 und der Detektoreinheit 25 relativ zum Untersuchungsbereich 21. Nach Erstellung eines oder mehrerer Schnittbilder kann der Vorschub der Transporteinrichtung 23 in z-Richtung um einen Schritt weiterlaufen. Die vorliegende Erfindung ist grundsätzlich bei beiden Abtastbewegungen gleichermaßen anwendbar und vorteilhaft.

Die von der Detektoreinheit 25 auf der rotierenden Gantry 3 akquirierten Messdaten, d.h. mittels der Detektorelemente 27 erfasster Intensitätswerte für erfasste Röntgenstrahlen, werden einem Datenverarbeitungssystem 31 zugeführt, das mit der Detektoreinheit 25, beispielsweise über nicht näher dargestellte Datenschleifringverbindung, und einer Steuereinheit 33 der Anlage 1 gekoppelt ist.

Die Röntgenprüfanlage 1 wird von der Steuereinheit 33 überwacht und gesteuert. Die Steuereinheit 33 ist über entsprechende Kommunikationsverbindungen mit allen Sensoren und Aktoren der Röntgenprüfanlage 1 verbunden, beispielsweise besteht ebenfalls eine kontaktlos arbeitende nicht näher dargestellte Datenschleifringverbindung zu den Strahlungsquellen 7, 9, die so im Betrieb von der Steuereinheit 33 selektiv aktiviert werden können. Ebenso ist die Steuereinheit 33 mit den Motoren 4 und 29 zum Antrieb der Gantry 3 bzw. der Transporteinrichtung 23 verbunden. Die Datenverarbeitungseinheit 31 ist ebenfalls mit der Steuereinheit 33 verbunden und empfängt u.a. für die Zuordnung der einzelnen durch die Detektionseinheit 25 erfassten Messwerte zu den einzelnen Strahlungsquellen 7, 9 notwendige Synchronisationssignale.

Die Datenverarbeitungseinheit 31 verarbeitet die erfassten Messwerte mit für den Einsatz in der Computertomografie bekannten Algorithmen, wobei beispielsweise eine zielgerichtete Auswertung die Detektion von in Inspektionsobjekten verborgenen Gefahrenstoffen, wie zum Beispiel Sprengstoffen, sein kann. Bei Bedarf kann die Datenverarbeitungseinrichtung 31 ein basierend auf den erfassten Messwerten rekonstruiertes 3D- oder 2D-Bild des Inneren des Inspektionsobjekts auf einem direkt oder indirekt (z.B. über ein Netzwerk) angeschlossenem Bildausgabeterminal 35 für einen Operator ausgeben.

Die Steuerung der Prüfanlage 1 durch die Steuereinheit 33 und die Auswertung erfasster Intensität der das Inspektionsobjekt durchdringenden Röntgenstrahlung durch das Datenverarbeitungssystem 31 sind Funktionen, die bekanntermaßen computerimplementiert werden können. Diese Funktionen können mittels separater Computersysteme vorgesehen sein bzw. auch durch ein einziges Computersystem bereitgestellt werden.

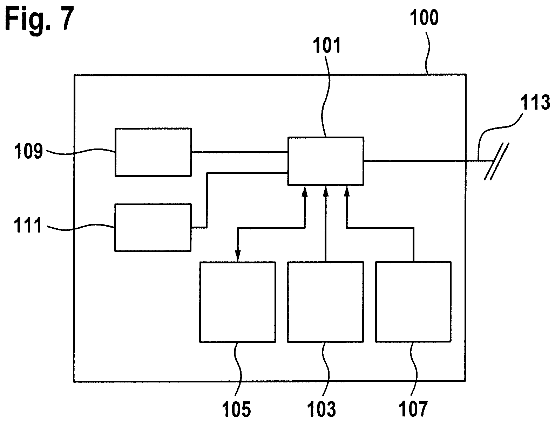

Figur 7 zeigt schematisch ein vereinfachtes Blockschaltbild eines Computersystems 100 zur Implementierung des Datenverarbeitungssystems 31 zur Verarbeitung erfasster Intensitätswerte für das Inspektionsobjekt durchdringender Röntgenstrahlen bzw. zur Implementierung der Steuereinheit 33. Es versteht sich, dass Datenverarbeitungssystems 31 und Steuereinheit 33 auch mittels eines einzigen Computersystems 100 implementiert werden können.

Es versteht sich auch, dass grundsätzlich das Datenverarbeitungssystem 31 zur Verarbeitung und Auswertung der durch die Röntgenprüfanlage 1 erfassten Intensitätswerte in dem Computersystem 100 erfolgen kann, welches lokal in oder an der Röntgenprüfanlage angeordnet ist. Es ist aber auch möglich, dass das Computersystem 100, beispielsweise über ein Computernetzwerk gekoppelt, an einem zentralen Ort nach Art eines Mainframe-Computersystems zentral, insbesondere für mehrere Röntgenprüfanlagen, angeordnet ist. Es ist auch möglich, dass das Computersystem 100 von mehreren miteinander über ein Computernetzwerk verbundenen und somit räumlich verteilten Computersystemen gebildet ist.

Daher zeigt die Figur 7 im Wesentlichen nur die grundsätzlichen Bestandteile des Computersystems 100, die zur Implementierung der Funktionen Steuerung und/oder Auswertung notwendig sind. Das Computersystem 100 besitzt wenigstens einen mittels Software steuerbaren Prozessor 101. In wenigstens einem ersten Speicher 103 sind Softwaremittel, beispielsweise Computerprogramme zur Umsetzung der gewünschten Funktionen abgelegt, wenn das Computerprogramm in dem wenigstens einen Prozessor 101 ausgeführt wird. Wenigstens ein zweiter Speicher 105 ist als Arbeitsspeicher für zu verarbeitende Daten und Zwischen- bzw. Endergebnisse vorgesehen. Ein dritter Speicher 107 kann vorgesehen sein, in dem Vergleichswerte für spezifische Materialgrößenwerte in Form einer Datenbank hinterlegt sind; dies können beispielsweise spezifische, die Absorption von Röntgenstrahlen beeinflussende Größen bekannter Materialen, insbesondere die Dichte und/oder der Massenschwächungskoeffizient und/oder die effektive Kernzahl dieser Materialien sein.

Weiter besitzt das Datenverarbeitungssystem 100 Eingabemittel 109, wie beispielsweise eine Tastatur, ein Touchpad, eine Zeigereingabeeinheit (Computermaus) oder dergleichen bzw. eine spezifisch auf eine einfache Bedienung abgestimmte Variante einzelner dieser Mittel bzw. Kombination davon. Zur Ausgabe von Meldungen bzw. Anzeigen sowie zur Visualisierung von einzelnen in einem Inspektionsobjekt enthaltenen Gegenständen bzw. seinem Inneren ist ein bildgebendes Ausgabemittel 111, wie beispielsweise wenigstens ein Bildschirm bzw. Monitor 35 (Figur 1) vorgesehen.

Über eine Datenschnittstelle 113 ist das Computersystem 100 mit der Röntgenprüfanlage 1, d.h. je nach zu implementierender Funktion mit Aktoren (wie Motoren 4, 29, Strahlungsquellen 7, 9 etc.) bzw. Sensoren (z.B. Detektoreinheit 25) gekoppelt.

Hinsichtlich des Computersystems 100 sei noch angemerkt, dass der Begriff "Prozessor" sich auf eine beliebige elektronische Einrichtung bzw. Schaltung oder einen Teil einer elektronischen Einrichtung bzw. Schaltung beziehen kann, welche(s) elektronische Daten aus Registern und/oder einem Speicher verarbeitet, um elektronische Daten in andere elektronische Daten umzusetzen bzw. aus Eingabedaten entsprechende Ausgabedaten zu erzeugen, die in Registern und/oder einem Speicher gespeichert werden können. Wie bereits angesprochen, kann das Computersystem 100 einen Prozessor 101 oder mehrere Prozessoren umfassen.

Das Computersystem 100 ist bevorzugt als Kombination aus Hardware, Firmware und Software realisiert. Entsprechend können die hier beschriebenen Verfahren teilweise oder auch vollständig als auf einem maschinenlesbaren Medium gespeicherte Softwaremittel realisiert werden, die zur Durchführung in das Computersystem 100 eingelesen und ausgeführt werden können. Ein maschinenlesbares Medium kann für einen beliebigen Mechanismus zum Speichern, Übertragen oder Empfangen von Information in einer von einem Computer lesbaren Form eingerichtet sein. Als nicht abschließende Beispiele seien hier Nur-Lese-Speicher (ROM), Direktzugriffsspeicher (RAM), Magnetspeicherplatten, optische Speichermedien, Flash-Speichermedien genannt. Schließlich können die Softwaremittel auch in Form eines Datenstroms als optische, akustische oder auf sonstige Art und Weise weitergeleitete Signale (z.B. als Trägerwellen, Infrarotsignale, digitale Signale usw.) vorliegen, die über entsprechende Schnittstellen, beispielsweise Antennen, die diese Signale aussenden und/oder empfangen, übertragen werden können.

Figur 2 zeigt eine Abwicklung einer ersten Ausführung der Detektoreinheit 25 in einer vereinfachten Darstellung, d. h., es sind deutlich weniger Zeilen 37 und Spalten 39 von Detektorelementen 27 als in der Realität angedeutet. Die einzelnen Detektorelement 27 sind jeweils vereinfacht durch ein Rechteck angedeutet. Das xyz-Koordinatensystem ist ebenfalls zur Orientierung und entsprechend zur Figur 1 dargestellt. Die Spalten 39 sind von der Mitte ausgehend nach außen positiv bzw. negativ nummeriert. Mit 42 ist die Gerade bezeichnet, die sich bei einer Projektion der Rotationsachse 5 von einem Punkt zwischen den Strahlungsquellen 7, 9 auf die Detektoreinheit 25 ergibt. Die Gerade 41 der Figur 1 ist ebenfalls zur Orientierung angedeutet.

Die Detektoreinheit 25 besteht nicht aus einem geometrisch vollständig (d.h. Füllfaktor 100%) durch Detektorelemente 27 besetzten Detektorfeld bzw. Detektorarray sondern aus zueinander durch Lücken 28 beabstandeten Detektorspalten 39. Die Breite der Lücken 28 entspricht dem Abstand D1 zwischen zwei benachbarten Detektorelementen 27. Die einzelnen Detektorelemente 27 besitzen eine Breite B. Der Abstand von einer rechten Kante K1 eines Detektorelements 27' zur rechten Kante K2 des unmittelbar benachbarten Detektorelementes 27" in derselben Zeile 37 ist mit P gekennzeichnet und wird mit dem engl. Fachausdruck Pitch bezeichnet. Der geometrische Wirkungsgrad (engl, geometric efficiency) bzw. die Zeilendichte der Detektoreinheit 25 ist als der Quotient B/P definiert.

Figur 3a ist eine schematische Draufsicht auf die Röntgenprüfanlage 1 der Figur 1 in Richtung z, d.h. in Richtung der Rotationsachse 5. Das xyz-Koordinatensystem ist ebenfalls zur Orientierung und entsprechend zur Figur 1 eingetragen. Am oberen Rand der Figur 3a sind auf der Umfanglinie der Gantry 3 die beiden Strahlungsquellen 7 und 9 gezeigt, die zueinander in einem Abstand D2 auf einer zur Rotationsachse 5 mit dem Radius R2 verlaufenden Kreisbahn angeordnet sind. Den Strahlungsquellen 7, 9 gegenüber angeordnet, ist die Detektoreinheit 25 mit den Detektorelementen 27 auf einer konzentrischen Kreisbahn mit Radius R1 angeordnet gezeigt. Im gezeigten Ausführungsbeispiel sind die Radien der Kreisbahnen R1, R2, auf denen sich die Strahlungsquellen 7, 9 bzw. die Detektoreinheit 25 bewegen gleich, d.h. es gilt R1 = R2.

Der zweite Abstand D2 zwischen den wenigstens zwei unabhängig ansteuerbaren Röntgenstrahlungsquellen 7, 9 ist im in der Figur 3a gezeigten Ausführungsbeispiel so eingestellt, dass gilt $D2 \approx B \leq D1$, wobei B die Breite eines Detektorelements 27 in Umfangsrichtung der Gantry 3 ist; d.h., die Breite D1 einer Lücke 28 ist mindestens so groß wie die Breite eines Detektorelements 27. In der Figur 3a sind die Abstände, besonders der Abstand D2, zur einfacheren Darstellung nicht maßstabsrichtig eingetragen.

Die Erfinder haben erkannt, dass es möglich ist, durch eine Erhöhung der Anzahl der rotierenden Strahlungsquellen um einen Faktor n (im hier besprochenen Ausführungsbeispiel n=2), die Anzahl der benötigten Detektorelemente 27 wenigstens entsprechend 1/n zu verringern (im Ausführungsbeispiel zu halbieren). Mit anderen Worten ist es möglich, durch eine Verdoppelung des Aufwandes bei der Strahlungsquelle, die Anzahl der benötigten Detektorelemente 27 mindestens zu halbieren.

Mit anderen Worten kann, wenn bei der gezeigten ersten Ausführung der zweite Abstand D2 der Strahlungsquellen 7, 9 auf etwa den Abstand der Breite B eines Detektorelements 27 eingestellt wird, wenigstens jede zweite Detektorzeile 29 wegfallen. D.h., der Pitch P ist dann genau das Doppelte der Breite B. Damit ergibt sich für den oben definierten geometrischen Wirkungsgrad 50% bzw. eine Detektorzeilendichte von 50%. Bei herkömmlichen gattungsgemäßen Röntgenanlagen mit einer Strahlungsquelle bzw. mehren Strahlungsquellen, die jedoch nicht erfindungsgemäß beabstandet sind, ist es zur Erzielung der gewünschten Auslösung des virtuellen Detektors im Messfeld erforderlich, die Detektoreinheit 25 mit Detektorelementen 27 derart zu besetzten, dass sich eine Detektorzeilendichte von annähernd 100% ergibt. Solche Detektoreinheiten sind komplex und aufwendig. Durch den erfindungsgemäßen Einsatz von wenigstens zwei entsprechend beabstandeten Strahlungsquellen 7, 9 werden zwischen den einzelnen Detektorspalten 39 mittels dem ersten Abstand D1 Lücken 28 geschaffen, deren Breite bevorzugt in einem Bereich von mehr als 100% bis maximal 250% der Breite B eines Detektorelements 27 eingestellt werden kann. Bereits bei einem Abstand D1, der gleich der Breite B eines Detektorelements 27 ist, halbiert sich der Aufwand hinsichtlich der Detektorelemente 27 und deren Verdrahtung, wobei im Wesentlichen die gleiche Auslösung im Messfeld erreicht wird, wie bei Anlagen mit einem Fokus und einer Detektoreinheit mit 100% Füllfaktor.

Die Erfinder haben weiter herausgefunden, dass der erste Abstand D1, d.h. die Breite der Lücke 28 zwischen den einzelnen Detektorzeilen 39 sogar — bei noch hinnehmbaren Qualitätsverlusten — auf den doppelten Wert der Breite B eines Detektorelements 27 erhöht werden kann. Für diesen Fall ergibt sich ein geometrischer Wirkungsgrad von 25% bzw. eine Detektorzeilendichte von 25%.

Ein Detektorelement 27 kann beispielsweise ein Szintillator-Pixel sein, das Röntgenstrahlung empfängt und in Lichtimpulse konvertiert, die dann über eine integrierte Photodiode in ein elektrisches Messsignal gewandelt werden können. Bei den herkömmlichen Detektoreinheiten 25 mit einem geometrischen Wirkungsgrad von nahezu 100% ist der Aufbau komplex, besonders ist die Verdrahtung der einzelnen Detektoren 27 kompliziert.

Figur 3b ist eine schematische Draufsicht auf die Röntgenprüfanlage 1 der Figur 1 in Richtung z, d.h. in Richtung der Rotationsachse 5, welche nicht Teil der Erfindung ist, mit einer alternativ ausgeführten Detektoreinheit 25', d.h. einer Abwandlung gegenüber der Figur 3a. Das xyz-Koordinatensystem ist ebenfalls zur Orientierung und entsprechend zur Figur 1 eingetragen. Am oberen Rand der Figur 3b sind auf der Umfanglinie der Gantry 3 die beiden Strahlungsquellen 7, 9 gezeigt, die zueinander in einem Abstand D2 auf einer zur Rotationsachse 5 mit dem Radius R2 verlaufenden Kreisbahn angeordnet sind. Den Strahlungsquellen 7, 9 gegenüber angeordnet, befindet sich die Detektoreinheit 25' mit den Detektorelementen 27, die auf einer Kreisbahn mit Radius R1' gegenüber ungefähr einem Punkt zwischen den Strahlungsquellen 7 und 9 angeordnet sind. Im gezeigten Ausführungsbeispiel gilt für die beiden Radien R1' und R2 der Kreisbahnen auf denen sich die Strahlungsquellen 7, 9 bewegen bzw. die Detektoreinheit 25 liegen ungefähr R1' = 2 R2, d.h. R1' ist ungefähr doppelt so groß wie R2.

Der zweite Abstand D2 ist im in der Figur 3b gezeigten Ausführungsbeispiel zwischen den wenigstens zwei unabhängig ansteuerbaren Röntgenstrahlungsquellen 7, 9 derart eingestellt ist, dass $D2 \approx B \leq D1$ gilt, wobei B die Breite eines Detektorelements 27 in Umfangsrichtung ist; auch bei Figur 3b sei angemerkt, dass die Abstände, besonders der Abstand D2, zur einfacheren Darstellung nicht maßstabsrichtig eingetragen ist.

Figur 4 zeigt Details der Detektoreinheit der Figur 3a mit aus Sicht der Strahlungsquellen 7, 9 ersten 27a und zweiten 27b übereinander angeordneten Detektorelementen um eine Dual-Energie- oder Mehrenergie-Auswertung zu ermöglichen. D.h., jedes Detektorelement 27 ist für die Erfassung von zwei Energiebereichen (bzw. Frequenzbereichen) aus dem Röntgenspektrum der Strahlungsquellen 7, 9 eingerichtet, indem die ersten Detektorelemente 27a zur Erfassung von Röntgenstrahlen aus dem ersten Energiebereich eingerichtet sind und die zweiten Detektorelemente 27b zur Erfassung von Röntgenstrahlen für einen zweiten Energiebereich eingerichtet sind. Dazu ist unter einem ersten Detektorelement 27a jeweils ein zweites Detektorelement 27b angeordnet.

Die ersten Detektorelemente 27a und die zweiten Detektorelemente 27b liefern jeweils einen zugehörigen Messwert, sodass jeweils Messwerte für zwei Energiebereiche vorliegen. Darauf basierend kann die Mehrenergieauswertung erfolgen; beispielsweise um neben einer wahrscheinlichen Dichte auch die wahrscheinliche effektive Ordnungszahl $Z_{eff}$ eines Materials in einem Raumelement (Voxel) des Untersuchungsbereichs bestimmen zu können. In der in der Figur 4 dargestellten Ausführung besteht ein Detektorelement 27 tatsächlich aus zwei übereinander angeordneten Detektoren, als Detektorelemente 27 können beispielsweise aus der o.g. EP 1 186 909 bekannte Detektorelemente verwendet werden.

Bei der erfindungsgemäßen Röntgenprüfanlage 1 können neben der Einsparung bei der Anzahl der benötigten Detektorelemente weitere Vorteile erreicht werden. Dadurch, dass die Detektoreinheit 25 im Wesentlichen nur noch aus zueinander beabstandeten Detektorspalten 39 aufgebaut ist, kann der Aufbau der Detektorelemente, besonders im Hinblick auf eine Mehrenergieauswertung weiter vereinfacht werden.

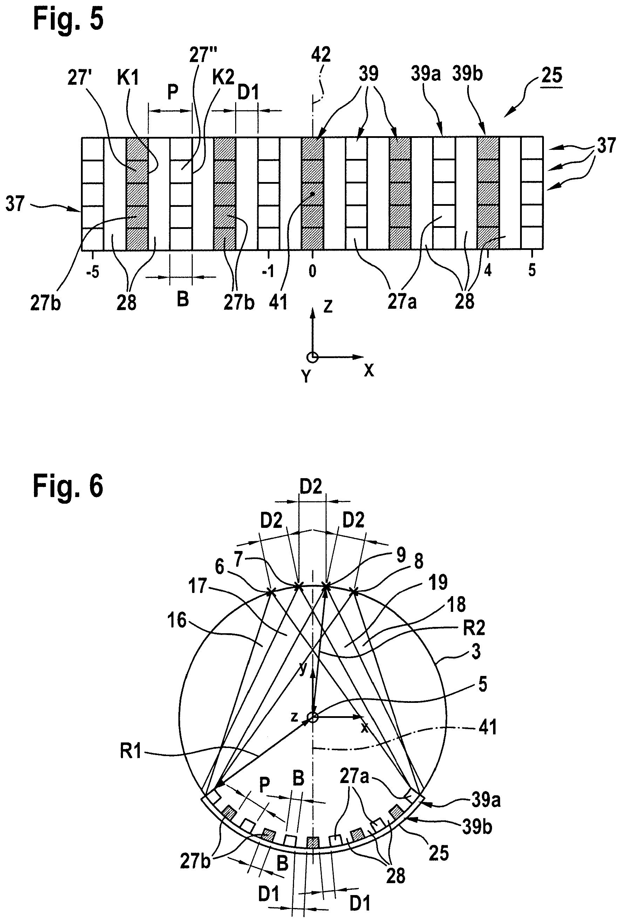

Figur 5 zeigt eine Draufsicht auf eine beispielhafte Alternative der Detektoreinheit 25 der Figur 4, die nicht Teil der Erfindung ist, mit ersten 27a und zweiten 27b nebeneinander angeordneten Detektorelementen für eine Mehrenergie-Auswertung. Figur 6 zeigt entsprechend eine schematische Draufsicht auf die Gantry 3 einer Weiterbildung der Röntgenprüfanlage der Figur 1 mit Blickrichtung entlang der Rotationsachse 5 mit der weitergebildeten Detektoreinheit 25 der Figur 5. Auch in dem Beispiel der Figuren 5 und 6 ist zwischen benachbarten Detektorelementen 27a, 27b eine Lücke 28 vorgesehen.

Bei den illustrativen Beispielen, welche nicht Teil der Erfindung sind, der Figuren 5 und 6 ist der tatsächliche Abstand zwischen zwei ersten Detektorelementen 27a oder zwei zweiten Detektorelementen 27b auf das 2-fache der Breite B eines der Detektorelemente 27a, 27b vergrößert; d.h., für das Verhältnis gilt $D1/B \approx 200\%$. Damit reduziert sich die Auflösung der mit den ersten bzw. zweiten Detektorelementen erfassten Informationen über das Inspektionsobjekt.

Wenn eine räumliche Auflösung wie bei der Ausführung der Figur 4 erreicht werden soll, kann dies entsprechend dem hier angewandten Prinzip durch eine entsprechende Erhöhung der Anzahl der Foci von n=2 auf n=4 erreicht werden. Zur Veranschaulichung dieser Ausführung sind in der Figur 6 zwei zusätzliche Strahlungsquellen 6 und 8 angedeutet, die jeweils links bzw. rechts im Abstand D2 zu jeweils einer der Strahlungsquellen 7 bzw. 9 angeordnet sind. Die Strahlungsquellen 6 und 8 sind ebenfalls jeweils eingerichtet ein Strahlenbündel 16 bzw. 18 mit auf die Detektoreinheit 25 ausgerichteten Röntgenstrahlen zu erzeugen.

Die ersten Detektorelemente 27a und zweiten Detektorelemente 27b sind in den Figuren 5 und 6 nicht wie in der Ausführung der Figur 4 übereinander, sondern nebeneinander angeordnet. Dadurch vereinfacht sich der Aufbau der Detektoreinheit 25 zusätzlich, besonders die Verdrahtung der einzelnen Detektorelemente 27 stellt keine Beeinträchtigung mehr dar, da keines der Detektorelemente 27 bei einer Mehrenergieauswertung im Strahlengang eines anderen Detektorelementes liegt.

Der erste Abstand D1 der einzelnen ersten 39a und zweiten 39b Detektorspalten ist hier ebenfalls auf mehr als die Breite B eines Detektorelementes 27a, 27b eingestellt (d.h. $D1/B \geq 100\%$). Damit steht auch bei dieser Ausführung jedem Detektorelement 27a, 27b wenigstens eine Lücke 28 zur Verfügung, die beispielsweise für eine vereinfachte Verdrahtung des Detektorelements 27a oder 27b genutzt werden kann.

Der Krümmungsradius der Detektoreinheiten 25 der Figuren 4 und 6 in Umfangsrichtung kann, wie in der Figur 3b gezeigt, entsprechend einer Kreisbahn mit dem Radius R1' um den Mittelpunkt der Foci ausgeführt sein. Bei dieser Ausführung erhöht sich zwar der Platzbedarf für die Detektoreinheit, dafür können jedoch alle der einzelnen Detektorelemente 27 gleichermaßen besser auf die Strahlungsquellen 7, 9 bzw. 6, 7, 8, 9 ausgerichtet werden.

Abschließend sei noch angemerkt, dass, auch wenn in den Ausführungen der Detektoreinheit 25 der Figuren 2 bis 6, eine bestimmte geometrische Lagebeziehung zwischen den Strahlungsquellen 7, 9 und den Detektorspalten 39 eingestellt ist, dies nicht zwingend ist. Die konkrete Lagebeziehung einer Ausführung muss lediglich in den Auswertealgorithmen berücksichtigt werden. Dabei führen Anordnungen bei der Lagebeziehung, bei denen mögliche Symmetrien ausgenutzt werden können zu Vereinfachungen bei den Algorithmen. Für das grundsätzliche Auswerteergebnis ist dies jedoch nicht wesentlich. D.h., die Vorteile der Erfindung werden bei jeder beliebig eingestellten geometrischen Lagebeziehung zwischen den Strahlungsquellen 7, 9 und den Detektorspalten 39 erreicht.

**Patentansprüche**

1. Röntgenprüfanlage (1) zur zerstörungsfreien Inspektion von Objekten mit

wenigstens zwei um eine Rotationsachse (5) in einer Rotationsebene drehbaren Röntgenstrahlungsquellen (7, 9) zum Erzeugen jeweils eines einen Untersuchungsbereich (21) durchsetzenden Strahlenbündels (17, 19), wobei eine Förderrichtung (z) für ein Inspektionsobjekt im Wesentlichen senkrecht zur Rotationsebene verläuft, und
einer den wenigstens zwei Röntgenstrahlungsquellen (7, 9) gegenüberliegend angeordneten Detektoreinheit (25) mit Detektorelementen (27; 27a, 27b) für Röntgenstrahlen, wobei sich jeweils zwischen in Umfangsrichtung der Rotationsebene benachbarten Detektorelementen (27; 27a, 27b), die mit einem ersten Abstand D1 beabstandet sind, eine Lücke (28) befindet, und ein Verhältnis D1/B des ersten Abstands D1 zu einer Breite B eines Detektorelements (27; 27a, 27b) mehr als 100% und höchstens 250% beträgt,

**dadurch gekennzeichnet, dass**

die Detektorelemente (27; 27a) erste Detektorelemente (27a) zur Detektion von Röntgenstrahlen eines ersten Energiebereichs und zweite Detektorelemente (27b) zur Detektion von Röntgenstrahlen eines zweiten Energiebereichs sind, wobei jeweils eines der zweiten Detektorelemente (27b) zur Detektion von Röntgenstrahlen des zweiten Energiebereichs unter jeweils einem der ersten Detektorelemente (27a) angeordnet ist, wobei jeweils ein erstes Detektorelement (27a) und ein zweites Detektorelement (27b) eine Einheit bilden und sich zwischen dieser Einheit und einer benachbarten Einheit die Lücke (28) befindet,

die Detektorelemente (27; 27a, 27b) in der Detektoreinheit (25) in Richtung der Rotationsachse (5) jeweils in Reihen mit mehreren Detektorelementen (27; 27a, 27b) angeordnet sind, wobei jede Reihe eine Detektorspalte (39; 39a, 39b) darstellt, und

die ersten Detektorelemente (27; 27a) auf einer ersten Kreisbahn mit einem ersten Radius R1' angeordnet sind, wobei die wenigstens zwei unabhängig ansteuerbaren Röntgenstrahlungsquellen (7, 9) in der Rotationsebene auf einer zweiten Kreisbahn mit einem zweiten Radius R2 um die Rotationsachse (5) und in der Rotationsebene in Umfangsrichtung mit einem zweiten Abstand D2 zueinander beabstandet angeordnet sind, wobei die erste Kreisbahn um einen Punkt auf der zweiten Kreisbahn mittig zu den wenigstens zwei unabhängig ansteuerbaren Röntgenstrahlungsquellen (7, 9) angeordnet ist, und wobei der zweite Abstand (D2) zwischen den wenigstens zwei unabhängig ansteuerbaren Röntgenstrahlungsquellen (7, 9) derart eingestellt ist, dass gilt

$$D2 \leq 2\frac{R1'+R2}{2R1'}B.$$

2. Röntgenprüfanlage (1) nach Anspruch 1, wobei das Verhältnis D1/B des ersten Abstands D1 zu der Breite B eines Detektorelements (27; 27a, 27b) in der Umfangsrichtung mindestens 110%, 125%, 150%, 175% oder 200% beträgt.

3. Röntgenprüfanlage (1) nach einem der vorhergehenden Ansprüche, weiter aufweisend eine Transporteinrichtung (23), welche zumindest teilweise in einer Einhausung der Röntgenprüfanlage (1) angeordnet und eingerichtet ist, ein Inspektionsobjekt durch die Röntgenprüfanlage (1) hindurch in der Förderrichtung (z) kontinuierlich oder schrittweise durch den Untersuchungsbereich (21) zu transportieren.

4. Röntgenprüfanlage (1) nach einem der vorhergehenden Ansprüche, weiter aufweisend einen feststehenden Teil, in dem drehbar um die Rotationsachse (5) eine Gantry (3) gelagert ist, wobei an der Gantry (3) wenigstens die wenigstens zwei Röntgenstrahlungsquellen (7, 9) in der Rotationsebene angeordnet sind, und wobei die Detektoreinheit (25) den wenigstens zwei Röntgenstrahlungsquellen (7, 9) gegenüberliegend (i) um die Rotationsachse (5) drehbar an der Gantry (3) oder (ii) den Untersuchungsbereich (21) in Umfangsrichtung umfassend an dem feststehenden Teil angeordnet ist.

5. Verfahren zur zerstörungsfreien Inspektion eines Objekts mit einer Röntgenprüfanlage (1) nach einem der Ansprüche 1 bis 4, aufweisend:

Drehen der wenigstens zwei unabhängig ansteuerbaren Röntgenstrahlungsquellen (7, 9) in der Rotationsebene um den Untersuchungsbereich (21);

abwechselndes Aktivieren jeder der wenigstens zwei unabhängig ansteuerbaren Röntgenstrahlungsquellen (7, 9) und dabei jeweils Bestrahlen von der den Röntgenstrahlungsquellen (7, 9) gegenüberliegend angeordneten Detektoreinheit (25) mit den ersten und zweiten Detektorelementen (27; 27a, 27b) durch den Untersuchungsbereich (17) hindurch;

Transportieren eines Inspektionsobjekts durch den Untersuchungsbereich (21); Erfassen und Auswerten der Intensitäten von das Inspektionsobjekt durchdringender Röntgenstrahlen mittels der ersten und zweiten Detektorelemente (27; 27a, 27b).

## Claims

1. X-ray inspection apparatus (1) for the non-destructive inspection of objects comprising

at least two X-ray radiation sources (7, 9) rotatable about an axis of rotation (5) in a plane of rotation for generating respectively a radiation beam (17, 19) passing through an inspection region (21), a conveying direction (z) for an inspection object running essentially perpendicular to the plane of rotation, and

a detector unit (25) arranged opposite the at least two X-ray radiation sources (7, 9) and having detector elements (27; 27a, 27b) for X-rays, with detector elements (27; 27a, 27b) which are spaced apart by a first distance D1, and a ratio D1/B of the first distance D1 to a width B of a detector element (27; 27a, 27b) is more than 100% and at most 250%,

**characterized in that**

the detector elements (27; 27a) are first detector elements (27a) for detecting X-rays of a first energy range and second detector elements (27b) for detecting X-rays of a second energy range, one of the second detector elements (27b) for detecting X-rays of the second energy range being respectively arranged below a corresponding one of the first detector elements (27a), wherein a first detector element (27a) and a second detector element (27b) respectively forming a unit, and the gap (28) being located between said unit and an adjacent unit

the detector elements (27; 27a, 27b) are arranged in the detector unit (25) in the direction of the axis of rotation (5) in respective rows with a plurality of detector elements (27; 27a, 27b), each row representing a detector column (39; 39a, 39b), and

the first detector elements (27; 27a) are arranged on a first circular path with a first radius R1', wherein the at least two independently controllable X-ray radiation sources (7, 9) are arranged in the plane of rotation on a second circular path with a second radius R2 around the axis of rotation (5) and are arranged in the plane of rotation at a distance from one another in the circumferential direction with a second distance D2 wherein the first circular path is arranged about a point on the second circular path centrally with respect to the at least two independently controllable X-ray radiation sources (7, 9), and wherein the second distance (D2) between the at least two independently drivable X-ray radiation sources (7, 9) is adjusted such that

$$D2 \leq 2\frac{R1'+R2}{2R1'}B.$$

2. X-ray inspection apparatus (1) according to claim 1, wherein the ratio D1/B of the first distance D1 to the width B of a detector element (27; 27a, 27b) in the circumferential direction is at least 110%, 125%, 150%, 175% or 200%.

3. X-ray inspection apparatus (1) according to any one of the preceding claims, further comprising a transport device (23) which is arranged at least partially in an enclosure of the X-ray inspection apparatus (1) and is arranged to transport an inspection object through the X-ray inspection apparatus (1) in the conveying direction (z) continuously or stepwise through the inspection area (21).

4. X-ray inspection apparatus (1) according to any one of the preceding claims, further comprising a stationary part in which a gantry (3) is mounted rotatably about the axis of rotation (5), wherein at least the at least two X-ray radiation sources (7, 9) are arranged on the gantry (3) in the plane of rotation, and wherein the detector unit (25) is arranged opposite the at least the two X-ray radiation sources (7, 9) (i) rotatably about the axis of rotation (5) on the gantry (3) or (ii) circumferentially encompassing the examination area (21) on the stationary part.

5. Method for non-destructive inspection of an object with an X-ray inspection apparatus (1) according to any of claims 1 to 4, comprising:

rotating the at least two independently controllable X-ray radiation sources (7, 9) in the plane of rotation around the inspection area (21);
alternately activating each of the at least two independently controllable X-ray sources (7, 9), thereby respectively irradiating the detector unit (25) arranged opposite the X-ray sources (7, 9) with the first and second detector elements (27; 27a, 27b) through the inspection area (17);
transporting an inspection object through the inspection area (21);
detecting and evaluating the intensities of X-rays penetrating the inspection object by means of the first and second detector elements (27; 27a, 27b).

**Revendications**

1. Installation de vérification aux rayons X convoyage (1) destinée à l'inspection non destructive d'objets, convoyage comprenant

EP 2 989 451 B1

au moins convoyage deux sources convoyage de rayonnement X (7, 9) pouvant tourner autour d'un convoyage axe de rotation (5) dans un plan de rotation pour convoyage générer respectivement un faisceau de rayons (17, 19) traversant une zone d'examen (21), cependant qu'un sens de convoyage convoyage convoyage (z) pour un objet d'inspection est essentiellement convoyage perpendiculaire au convoyage plan de rotation, et une unité de détecteur convoyage (25) dotée d'éléments convoyage de détecteur convoyage (27; 27a, 27b) pour rayons X, agencée convoyage à l'opposé des convoyage au moins convoyage deux sources convoyage de rayonnement X (7, 9), cependant que, respectivement entre des éléments convoyage de détecteur convoyage (27; 27a, 27b) avoisinants dans le sens circonférentiel de l'axe de rotation et espacés d'un premier espacement D1, il y a un écart (28), et un rapport D1/B entre le premier espacement D1 et une largeur B d'un convoyage élément de détecteur convoyage (27; 27a, 27b) est supérieur à 100 % et s'élève au maximum à 250 %, **caractérisé en ce que**
les éléments convoyage de détecteur convoyage (27; 27a) convoyage sont des premiers convoyage éléments convoyage de détecteur convoyage (27a) pour la détection de rayons X d'une première plage d'énergie et des deuxièmes convoyage convoyage éléments convoyage de détecteur convoyage (27b) pour la détection de rayons X d'une deuxième plage d'énergie, convoyage cependant que respectivement un des convoyage deuxièmes convoyage convoyage éléments convoyage de détecteur convoyage (27b) est, convoyage pour la détection de rayons X de la deuxième plage d'énergie, agencé sous convoyage respectivement un des premiers convoyage éléments convoyage de détecteur convoyage (27a), cependant que respectivement un premier convoyage élément convoyage de détecteur convoyage (27a) et un deuxième convoyage convoyage élément convoyage de détecteur convoyage (27b) constituent une unité et que, entre cette convoyage unité et une convoyage unité convoyage avoisinante, il y a l'écart (28),
les éléments convoyage de détecteur convoyage (27; 27a, 27b) convoyage convoyage sont agencés dans l'unité de détecteur convoyage (25) dans le sens de l'axe de rotation (5) respectivement en rangées de plusieurs éléments convoyage de détecteur convoyage (27; 27a, 27b), cependant que chaque rangée représente une fente de détecteur convoyage convoyage (39; 39a, 39b), et
les premiers convoyage éléments convoyage de détecteur convoyage (27; 27a) convoyage sont agencés sur une première trajectoire circulaire convoyage ayant un premier rayon R1', convoyage cependant que les convoyage au moins convoyage deux sources convoyage de rayonnement X (7, 9) pouvant être commandées indépendamment convoyage sont, dans le plan de rotation, agencées sur une deuxième trajectoire circulaire convoyage ayant un deuxième rayon R2 convoyage autour de l'axe de rotation (5), et sont, convoyage dans le plan de rotation, espacées entre elles dans le sens circonférentiel d'une deuxième convoyage espacement D2, cependant que convoyage la première trajectoire circulaire est agencée autour d'un point sur la convoyage deuxième trajectoire circulaire convoyage centré entre les au moins convoyage deux sources convoyage de rayonnement X (7, 9) pouvant être commandées indépendamment, et cependant que convoyage le convoyage deuxième convoyage espacement D2 convoyage entre les au moins convoyage deux sources convoyage de rayonnement X (7, 9) pouvant être commandées indépendamment est réglé de telle façon qu'il est entendu que :

$$D2 \leq 2\frac{R1'+R2}{2R1'}\times B.$$

2. Installation de vérification aux rayons X convoyage (1) selon la revendication 1, convoyage cependant que convoyage le rapport D1/B entre le premier espacement D1 et la largeur B d'un convoyage élément de détecteur convoyage (27; 27a, 27b) dans le sens circonférentiel s'élève au moins à 110 %, convoyage 125 %, 150 %, 175 % ou 200 %.

3. Installation de vérification aux rayons X convoyage (1) selon une des revendications précédentes, comportant en outre un équipement convoyage de convoyage convoyage convoyage (23) agencé au moins partiellement dans un boîtier de l'installation de vérification aux rayons X convoyage (1) et conçu pour transporter en continu ou par étapes un convoyage objet d'inspection convoyage à travers l'installation de vérification aux rayons X (1) dans le sens de convoyage convoyage (z) en passant dans la zone convoyage d'examen (21).

4. Installation de vérification aux rayons X convoyage (1) selon une des revendications précédentes, comportant en outre une partie fixe dans laquelle un portique (3) est convoyage logé de manière à pouvoir tourner autour de l'axe de rotation (5), cependant que, au convoyage portique (3), convoyage au moins convoyage les convoyage au moins convoyage deux sources convoyage de rayonnement X (7, 9) sont agencées dans le plan de rotation, et cependant que l'unité de détecteur convoyage (25) est agencée convoyage au convoyage portique (3) à l'opposé convoyage (i) des au moins convoyage deux sources convoyage de rayonnement X (7, 9) de manière à pouvoir tourner convoyage autour de l'axe de rotation (5), ou (ii) à la partie fixe en comprenant convoyage dans le sens circonférentiel la zone convoyage d'examen (21).

5. Procédé d'inspection non destructive d'un objet avec une installation de vérification aux rayons X convoyage (1) convoyage selon une des revendications de 1 à 4, comportant :

mise en rotation des convoyage au moins convoyage deux sources convoyage de rayonnement X (7, 9) pouvant être commandées indépendamment convoyage dans le plan de rotation convoyage autour de la zone convoyage d'examen (21) ;
activation alternative de chacune des au moins convoyage convoyage deux sources convoyage de rayonnement X (7, 9) pouvant être commandées indépendamment, tout en irradiant respectivement l'unité de détecteur convoyage (25) agencée à l'opposé des convoyage sources convoyage de rayonnement X (7, 9) avec les premiers et deuxièmes convoyage convoyage éléments convoyage de détecteur convoyage (27; 27a, 27b) à travers la zone d'examen (17) ;
transport d'un objet d'inspection convoyage en passant dans convoyage la zone d'examen (21);

saisie et évaluation, au moyen convoyage convoyage des premiers et deuxièmes convoyage convoyage éléments convoyage de détecteur convoyage (27; 27a, 27b), des intensités de rayons X pénétrant l'objet d'inspection.

# Fig. 1

## Fig. 2

## Fig. 3a

EP 2 989 451 B1

## Fig. 3b

## Fig. 4

18

## Fig. 5

## Fig. 6

**Fig. 7**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10149254 A1
- US 7362847 B2
- US 5361291 A
- EP 0231037 A1
- US 7778383 B2
- WO 2007110795 A2
- WO 2012112153 A
- WO 2012112153 A1
- WO 2013126649 A2
- US 5841832 A
- WO 2006114716 A2
- EP 1186909 A